# EUROPÄISCHE PATENTSCHRIFT

(11) **EP 2 182 901 B1**
(45) Veröffentlichungstag und Bekanntmachung des Hinweises auf die Patenterteilung: **31.12.2014**
(21) Anmeldenummer: 08801720.7
(22) Anmeldetag: 27.08.2008
(51) Int. Cl.: A61F 11/08

(54) **GEHÖRSCHUTZSTÖPSEL**
EAR PLUG
BOUCHON D'OREILLE

(30) Priorität: 07.09.2007 DE 102007042740
(43) Veröffentlichungstag der Anmeldung: 12.05.2010
(73) Patentinhaber: Uvex Arbeitsschutz GmbH, 90766 Fürth (DE)
(72) Erfinder: BERG, Göran, S-282 33 Tyringe (SE); HAKANSSON, Jörgen, S-282 31 Tyringe (SE)
(74) Vertreter: Bauerschmidt, Peter
(86) Internationale Anmeldenummer: PCT/EP2008/006991
(87) Internationale Veröffentlichungsnummer: WO 2009/033565

(56) Entgegenhaltungen:
- WO-A-01/76520
- WO-A-98/25558
- DE-U1- 20 200 638
- US-A- 5 488 961

## Beschreibung

Die Erfindung betrifft einen Gehörschutzstöpsel bestehend aus einem Grundkörper aus Schaumstoff, wobei der Grundkörper eine zentrale Mittenlängsachse sowie in Richtung dieser zentralen Mittenlängsachse eine axial innere Stirnseite und eine axial äußere Stirnseite hat.

Herkömmliche derartige Gehörschutzstöpsel sind so ausgelegt, dass sie den Gehörgang möglichst vollständig verschließen, um auf diese Weise eine maximale Schalldämmung zu erreichen.

Es gibt jedoch zahlreiche Anwendungsbereiche, wo es zwar erforderlich und wünschenswert ist, eine Schallreduzierung zu erreichen, wo es aber gleichwohl wichtig ist, eine Verständigungsmöglichkeit über Sprache aufrechtzuerhalten oder Umgebungsgeräusche oder Warnsignale wahrzunehmen.

Ein weiteres Problem bei herkömmlichen Gehörschutzstöpseln besteht darin, dass sie Störgeräusche generieren, wobei es sich insbesondere um otoakustische Emissionen, Knochenschallübertragungen oder eine Übertragung der eigenen Sprache ins Ohr handelt.

Solche otoakustischen Emissionen sind Töne, die im Ohr selbst erzeugt werden und dementsprechend praktisch aus dem Ohr herauskommen. Die Emission erfolgt spontan oder auf einen externen akustischen Reiz hin. Diese Töne werden von den Haarzellen des Corti-Organs in der Cochlea oder Schnecke erzeugt und entgegen der primären Flussrichtung des Schalls, die in das Ohr hinein gerichtet ist, retrograd emittiert. Dies bedeutet, dass die Emission das Corti-Organ über das ovale Fenster verlässt und damit auch das Innenohr, um dann über die Gehörknöchelchen das Mittelohr zu passieren und über das Trommelfell in den äußeren Gehörgang zu gelangen, wo sie dann über sehr empfindliche Messmikrofone auch nachweisbar sind.

Knochenleitung, auch Knochenschall genannt, bezeichnet die Weiterleitung von Schall-Schwingungen bzw. Vibrationen durch die das Gehörorgan umgebende Knochensubstanz unter Umgehung des Mittelohrs. Die bewusste Wahrnehmung des Knochenschalls wird wegen des hohen Schallwellenwiderstandes des Schädelknochens in der Regel von den als Luftschall übertragenen Signalen überdeckt. Bei der Verwendung von Gehörschutz führt der Knochenschall zu einer Klangverfälschung.

Dieser sogenannte Occlusionseffekt, der neben der Knochenleitung auch auf der Gewebeschallleitung beruht, führt dazu, dass der Benutzer die eigene Stimme nicht nur verfälscht sondern auch verstärkt hört ebenso wie andere Körpergeräusche, wie z. B. Atmung und Blutströmung, was dazu führt, dass eine gewisse Blockade beim Benutzer zu beobachten ist bzw. eine Isolierung.

In der DE 20 2005 009 132 U, der WO 2005/122981 A1 und in der WO 2004/064672 A2 wird jeweils ein Gehörschutzstöpsel beschrieben, der an einer seiner axialen Stirnseiten eine Ausnehmung hat. Ein weiterer in der DE 202 00 638 U1 beschriebener Gehörschutzstöpsel ist im Wesentlichen hohl ausgebildet und an einer axialen Stirnseite mit einem Diaphragma versehen. Aus der DE 93 02 783 U1, der DE 73 14 145 U1, der DE 690 13 927 T2 und der US 6,484,842 B1 sind zum Teil mehrteilige Gehörschutzstöpsel mit jeweils axial durchgehender Längsausnehmung, die in Längsrichtung variierende Öffnungsweiten aufweisen kann, bekannt.

Weiterhin sind in der DE 690 13 927 T2, der US 1,016,877 und der US 6,484,842 B1 Gehörschutzstöpsel offenbart, die an ihrer seitlichen Mantefläche mit Ausnehmungen versehen sind.

Der nächstliegende Stand der Technik WO-A-01/76520 offenbart die technischen Merkmale des Oberbegriffs aus Anspruch 1.

Hiervon ausgehend liegt der Erfindung die Aufgabe zugrunde, einen bekannten Gehörschutzstöpsel der gattungsgemäßen Art so weiterzubilden, dass die Schallabsorption bzw. -durchlässigkeit definiert einstellbar ist, und dass die Entstehung von Störgeräuschen vermieden bzw. reduziert wird.

Diese Aufgabe wird durch einen Gehörschutzstöpsel entsprechend den Merkmalen des Anspruchs 1 gelöst. Bei dem erfindungsgemäßen Gehörschutzstöpsel sind im Bereich der inneren Stirnseite oberflächenvergrößernde innere Ausnehmungen vorgesehen, von denen sich eine als eine innere Zentrallängsausnehmung ausgehend von der inneren Stirnseite längs der Mittenlängsachse nach innen erstreckt. Weiterhin ist eine äußere Zentrallängsausnehmung vorgesehen, welche sich ausgehend von der äußeren Stirnseite längs der Mittenlängsachse nach innen erstreckt. Die innere und die äußere Zentrallängsausnehmung sind durch eine senkrecht zur zentralen Mittenlängsachse verlaufende durchgehende Querwand voneinander getrennt.

Die inneren Ausnehmungen bilden insbesondere Kavitäten, wodurch die Oberfläche am inneren Ende des Gehörschutzstöpsels vergrößert wird. Diese günstige Vergrößerung betrifft insbesondere diejenige Oberfläche, die sich im Wesentlichen quer zum Gehörgang erstreckt, und auf die die endogen erzeugten Schallwellen auftreffen. Durch diese Oberflächenvergrößerung wird im Gegensatz zu einer kugelkalottenförmigen Oberfläche bei herkömmlichen Gehörschutzstöpseln die Reflexion der Schallwellen vermindert und insbesondere eine gerichtete Reflexion vermieden.

In der Anfangszeit der Entwicklung von Gehörschutzstöpseln wurden diese aus Mineralwolle gefertigt, welche an der inneren Stirnseite aufgrund der Struktur der Mineralwolle auch gewisse Ausnehmungen besaß. Anders als ein erfindungsgemäßer Gehörschutzstöpsel aus Schaumstoff wiesen diese damals bekannten Produkte aber insgesamt eine schlechte Schalldämmung auf.

Als innere Ausnehmungen bzw. Kavitäten kommen die unterschiedlichsten Strukturen in Betracht, wie z. B. golfballartige Strukturen oder Strukturen nach Art eines Eierkartons. Es wäre auch denkbar, die Spitze eines Ohrstöpsels nach dem (Spritz-)Gießen einfach abzuschneiden, so dass die vergleichsweise geschlossene Gussoberfläche teilweise wieder entfernt wird und an der inneren Stirnseite eine ansonsten nur im Inneren des Grundkörpers gegebene offenporige Struktur des Schaumstoffmaterials vorhanden ist. Auch diese dann stirnseitig vorhandenen offenen Poren sind erfindungsgemäße Ausnehmungen, die die akustisch vorteilhafte Oberflächenvergrößerung bewirken.

Zusätzlich erweist es sich als Vorteil, die sich in das Innere hinein erstreckende Zentrallängsausnehmung vorzusehen, wobei sich mit dieser fluchtend von der äußeren Stirnseite her eine zweite Zentrallängsausnehmung in das Innere hinein erstreckt und die beiden Zentrallängsausnehmungen aber durch eine Querwand voneinander getrennt sind.

Durch diese beiden Zentrallängsausnehmungen wird die effektive Materialdicke des Gehörschutzstöpsels vermindert, wodurch die Durchlässigkeit, z. B. für eine Sprachverständigung, definiert erhöht wird. Der erfindungsgemäße Gehörschutzstöpsel ist also teilweise schalldurchlässig. Insbesondere ist er so ausgelegt, dass Nutzschall in gewissem Umfang passieren kann. Die im Inneren vorgesehene Querwand entkoppelt die beiden Zentrallängsausnehmungen akustisch voneinander. Starke Außengeräusche werden dadurch gedämmt und gelangen nicht in das Innere des Ohrs. Außerdem wird eine unerwünschte akustische Mitkopplung der in der inneren und in der äußeren Zentrallängsausnehmung vorhandenen Schalldruckpegel verhindert. Weiterhin bietet die Querwand auch einen fertigungstechnologischen Vorteil. Sie stellt nämlich sicher, dass beim Spritzgießen der Gehörschutzstöpsel ein Auswerfen mittels Druckluft erfolgen kann. Trotz der erfindungsgemäß vorgesehenen Materialschwächung ist der Gehörschutzstöpsel insgesamt noch so stabil, dass er definiert und problemlos in den Gehörgang eingesetzt werden kann. Darüber hinaus hat die innere Zentrallängsausnehmung wie vorstehend bereits erläutert auch eine vorteilhafte Wirkung bei der Schwächung der Schallreflexion in das Innere des Ohres zurück.

Es ist vorgesehen, dass die oberflächenvergrößernden inneren Ausnehmungen auch Mantelausnehmungen zumindest teil- oder bereichsweise mit umfassen. Diese Mantelausnehmungen sind an einer Mantelfläche des Grundkörpers angeordnet und verlaufen in Richtung der zentralen Mittenlängsachse. Sie sind außerdem vorzugsweise rinnenförmig ausgestaltet und erstrecken sich ausgehend von einem seitlichen Bereich der Mantelfläche insbesondere bis in die Nähe einer an der inneren Stirnseite angeordneten Öffnung der inneren Zentrallängsausnehmung. Mit ihren an oder in die innere Stirnseite reichenden Teilbereichen tragen sie ebenfalls zur Verringerung der bei konventionellen Gehörschutzstöpseln an der inneren Stirnseite auftretenden, gegebenenfalls gerichteten inneren Schallwellenreflexionen bei.

Durch diese Ausgestaltung wird außerdem erreicht, dass der Gehörschutzstöpsel einen guten Sitz ohne Ausübung von Druck auf die Innenseite des Gehörkanals aufweist. Der gute Sitz wird auch dadurch erreicht, dass der Stöpsel zur Reduzierung des Dämpfungsverhaltens und zur Einstellung einer definierten Dämpfung nicht verkürzt werden muss.

Mit Vorteil können beispielsweise vier solche gleichmäßig um die Mantelfläche herum verteilte Mantelausnehmungen vorgesehen sein. Es entsteht hierdurch in der Aufsicht eine Art sternenförmige Konfiguration.

In weiterer Ausgestaltung der Erfindung kann vorgesehen sein, dass das stirnseitige äußere Ende des Gehörschutzstöpsels eine kragenartige Verbreiterung aufweist. Die äußere Stirnseite kann mit wenigstens einer Basisausnehmung versehen sein, in deren Basisboden die äußere Zentrallängsausnehmung mündet. Dadurch lässt sich der Gehörschutzstöpsel vor dem Einsetzen in das Ohr besser zusammendrücken. Dies erleichtert den Gebrauch.

Nachfolgend wird die Erfindung anhand eines bevorzugten Ausführungsbeispiels in Verbindung mit der Zeichnung näher erläutert. Dabei zeigen:
- Fig. 1: ein Ausführungsbeispiel eines Gehörschutzstöpsels in einer perspektivischen Darstellung aus einem schrägen Blickwinkel auf die im Gebrauchszustand äußere Stirnseite,
- Fig. 2: den Gehörschutzstöpsel gemäß Fig. 1 in einer perspektivischen Darstellung aus einem schrägen Blickwinkel auf die im Gebrauchszustand innere Stirnseite,
- Fig. 3: den Gehörschutzstöpsel gemäß Fig. 1 und 2 in einer Draufsicht auf die im Gebrauchszustand innere Stirnseite,
- Fig. 4: den Gehörschutzstöpsel gemäß Fig. 1 bis 3 in einer Draufsicht auf die im Gebrauchszustand äußere Stirnseite, und
- Fig. 5: den Gehörschutzstöpsel gemäß Fig. 1 bis 4 in einer Seitenansicht.

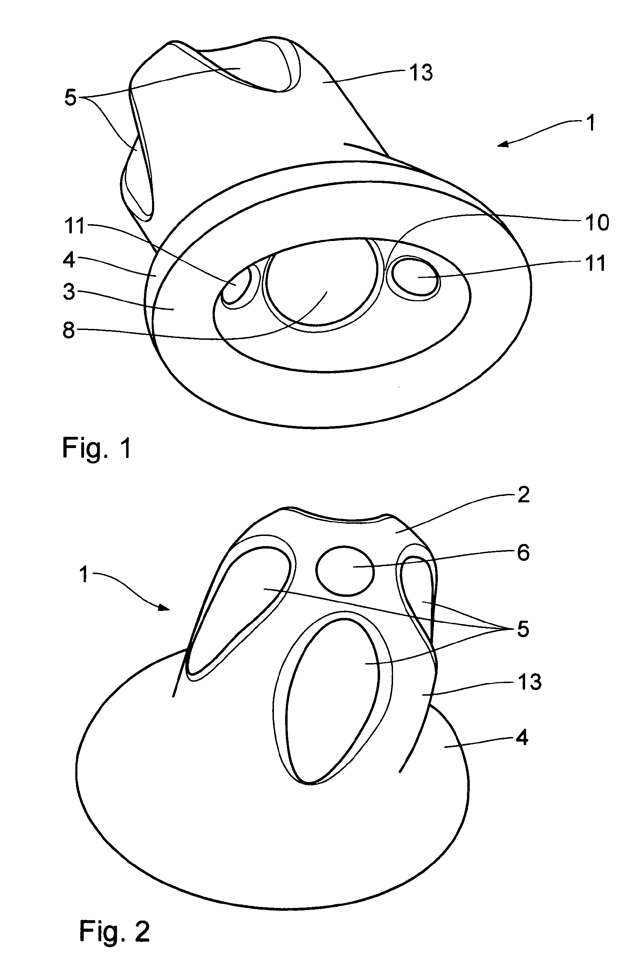

Ein in Fig. 1 bis 5 gezeigtes Ausführungsbeispiel eines erfindungsgemäßen einstückigen Gehörschutzstöpsels besteht aus Schaumstoff und weist einen annähernd glockenförmigen Grundkörper 1 auf. Er hat eine innere Stirnseite 2, welche im Tragezustand in Richtung auf das Innere des Gehörgangs zu in das Ohr eingesetzt wird, und eine äußere Stirnseite 3, welche im Tragezustand nach außen gewandt ist. Der Grundkörper 1 mündet mittels einer kragenartigen Verbreiterung 4 in die äußere Stirnseite 3.

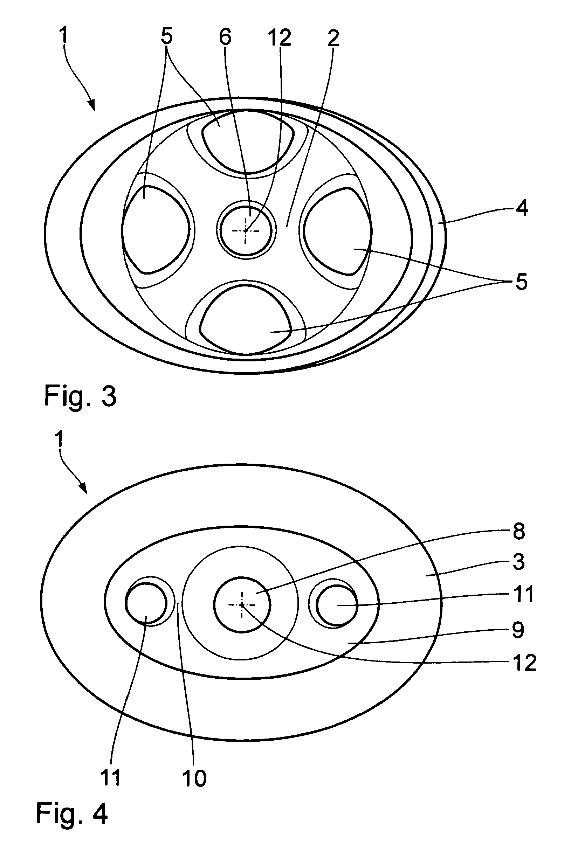

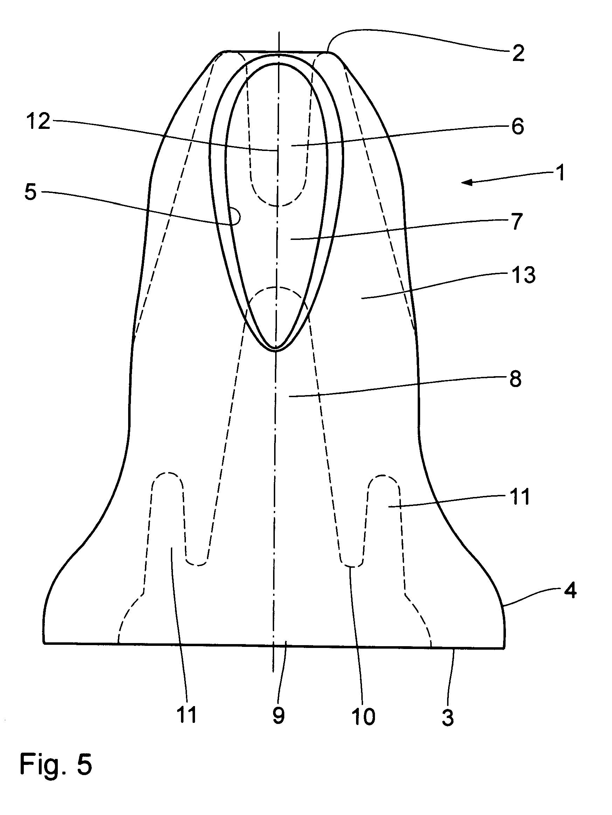

Der Grundkörper 1 hat eine zentrale Mittenlängsachse 12, deren axiale Richtung im Wesentlichen auch der Einsteckrichtung des Gehörschutzstöpsels entspricht, sowie eine Mantelfläche 13. Er ist an seiner Mantelfläche 13 mit in Längsrichtung, also in Richtung der zentralen Mittenlängsachse 12, verlaufenden rinnenartigen Mantelausnehmungen 5 versehen, welche sich bis in die innere Stirnseite 2 hinein erstrecken und zu einer Strukturierung derselben und insbesondere zu einer Ausbildung von Kavitäten führen, wie dies in Fig. 3 besonders gut erkennbar ist. Die Mantelausnehmungen 5 sind in der Aufsicht näherungsweise oval (siehe Fig. 2 und 5).

Vom Zentrum der inneren Stirnseite 2 erstreckt sich eine zylinderförmige bzw. eine leicht konische Zentrallängsausnehmung 6 längs der Mittenlängsachse 12 nach innen in den Grundkörper 1 hinein. Sie reicht bis zu einer Querwand 7, von deren von der inneren Zentrallängsausnehmung 6 abgewandten Seite sich eine ebenfalls zylinderförmige oder leicht konische äußere Zentrallängsausnehmung 8 nach außen bis zu einer verbreiterten, topfartigen Basisausnehmung 9, welche das Zentrum der äußeren Stirnseite 3 einnimmt, erstreckt. Wenn für die innere und/oder äußere Zentrallängsausnehmung 6 bzw. 8 eine konische Form vorgesehen ist, ist diese so ausgestaltet, dass sich ihre Öffnungsweite zu der jeweiligen Stirnseite 2 bzw. 3 hin vergrößert bzw. zur Querwand 7 hin verkleinert. Auch die äußere Zentrallängsausnehmung 8 verläuft längs der Mittenlängsachse 12. Die durchgehende Querwand 7 verläuft senkrecht zur Mittenlängsachse 12. Sie hat keine Längsdurchbrechung bzw. keinen Verbindungskanal in Richtung der Mittenlängsachse 12, so dass sie die innere und die äußere Zentrallängsausnehmung 6 bzw. 8 insofern vollständig voneinander trennt. Die Querwand 7 ist also auch eine Trennwand.

Bei dem erfindungsgemäßen Gehörschutzstöpsel ist es zur Erzielung einer definierten Dämpfung und zur Vermeidung von Störungen durch Eigengeräusche vorgesehen, dass die Oberfläche des Gehörschutzstöpsels im Bereich der inneren Stirnseite 2 mit den oberflächenvergrößernden Ausnehmungen, nämlich mit der inneren Zentrallängsausnehmung 6 und zumindest teilweise mit den Mantelausnehmungen 5, versehen ist.

Seitlich neben der äußeren Zentrallängsausnehmung 8 sind zwei weitere zylinderförmige Befestigungsausnehmungen 11 angeordnet. Sie erstrecken sich ausgehend von einem Basisboden 10 der Basisausnehmung 9 parallel zur Zentrallängsausnehmung 8 und parallel zur Mittenlängsachse 12 nach innen in den Grundkörper 1. Sie dienen zur Aufnahme eines Befestigungsendabschnitts einer Verbindungskordel, mittels derer zwei Gehörschutzstöpsel miteinander verbunden werden können. Ein Benutzer kann dann die Verbindungskordel mit den daran angebrachten Gehörschutzstöpseln um seinen Hals legen, so dass die Gehörschutzstöpsel jederzeit griffbereit sind.

## Patentansprüche

1. Gehörschutzstöpsel bestehend aus einem Grundkörper (1) aus Schaumstoff, wobei
a) der Grundkörper (1) eine zentrale Mittenlängsachse (12) sowie in Richtung dieser zentralen Mittenlängsachse (12) eine axial innere Stirnseite (2) und eine axial äußere Stirnseite (3) hat,
b) im Bereich der inneren Stirnseite (2) oberflächenvergrößernde innere Ausnehmungen (5, 6) vorgesehen sind, von denen sich eine als eine innere Zentrallängsausnehmung (6) ausgehend von der inneren Stirnseite (2) längs der Mittenlängsachse (12) nach innen erstreckt,
c) eine äußere Zentrallängsausnehmung (8) vorgesehen ist, welche sich ausgehend von der äußeren Stirnseite (3) längs der Mittenlängsachse (12) nach innen erstreckt,
d) die innere und die äußere Zentrallängsausnehmung (6, 8) durch eine senkrecht zur zentralen Mittenlängsachse (12) verlaufende durchgehende Querwand (7) voneinander getrennt sind, **dadurch gekennzeichnet, dass**
e) die oberflächenvergrößernden inneren Ausnehmungen (5, 6) an einer Mantelfläche (13) des Grundkörpers (1) angeordnete und in Richtung der zentralen Mittenlängsachse (12) verlaufende Mantelausnehmungen (5) zumindest teil- oder bereichsweise mit umfassen.

2. Gehörschutzstöpsel nach Anspruch 1, **dadurch gekennzeichnet, dass** vier gleichmäßig um die Mantelfläche (13) herum verteilte Mantelausnehmungen (5) vorgesehen sind.

3. Gehörschutzstöpsel nach Anspruch 1, **dadurch gekennzeichnet, dass** die Mantelausnehmungen (5) rinnenförmig sind und sich ausgehend von einem seitlichen Bereich der Mantelfläche (13) bis in die Nähe einer an der inneren Stirnseite (2) angeordneten Öffnung der inneren Zentrallängsausnehmung (6) erstrecken.

4. Gehörschutzstöpsel nach Anspruch 1, **dadurch gekennzeichnet, dass** der Gehörschutzstöpsel einstückig ausgebildet ist,

5. Gehörschutzstöpsel nach Anspruch 1, **dadurch gekennzeichnet, dass** im Bereich der äußeren Stirnseite (3) eine kragenartige Verbreiterung (4) vorgesehen ist.

6. Gehörschutzstöpsel nach Anspruch 1, **dadurch gekennzeichnet, dass** an der äußeren Stirnseite (3) eine Basisausnehmung (9) vorgesehen ist, in deren Basisboden (10) die äußere Zentrallängsausnehmung (8) mündet.

7. Gehörschutzstöpsel nach Anspruch 1, **dadurch gekennzeichnet, dass** seitlich neben der äußeren Zentrallängsausnehmung (8) mindestens eine weitere Längsausnehmung (11) vorgesehen ist, die sich parallel zur äußeren Zentrallängsausnehmung (8) und zur zentralen Mittenlängsachse (12) in den Grundkörper (1) hinein erstreckt.

## Claims

1. A hearing protection earplug consisting of a main body (1) consisting of foamed material, wherein
a) the main body (1) has a central center longitudinal axis (12) and in the direction of this central center longitudinal axis (12) an axially inner end face (2) and an axially outer end face (3),
b) surface area enlarging inner hollows (5, 6) are provided in the area of the inner end face (2), one of which, in the form of an inner central longitudinal hollow (6), extends inward longitudinally relative to the center longitudinal axis (12), starting from the inner end face (2),
c) an outer central longitudinal hollow (8) is provided that extends inward longitudinally relative to the center longitudinal axis (12), starting from the outer end face (3),
d) the inner and the outer central longitudinal hollow (6, 8) are separated from each other by a continuous transverse wall (7) extending perpendicular relative to the central center longitudinal axis (12),
**characterized in that**
e) the surface area enlarging inner hollows (5, 6) at least partly or in some areas also incorporate circumferential-surface hollows (5) disposed at a circumferential surface (13) of the main body (1) and extending in the direction of the central center longitudinal axis (12).

2. A hearing protection earplug according to claim 1 **characterized in that** four circumferential-surface hollows (5) are provided that are evenly distributed about the circumferential surface (13).

3. A hearing protection earplug according to claim 1, **characterized in that** the circumferential-surface hollows (5) are channel-shaped and extend starting from a lateral area of the circumferential surface (13) to within the vicinity of an opening of the inner central longitudinal hollow (6) disposed at the inner end face (2).

4. A hearing protection earplug according to claim 1, **characterized in that** the hearing protection earplug is formed in one piece.

5. A hearing protection earplug according to claim 1, **characterized in that**, in the area of the outer end face (3), a collar-like widening (4) is provided.

6. A hearing protection earplug according to claim 1, **characterized in that**, at the outer end face (3) a base hollow (9) is provided, into the base bottom (10) of which the outer central longitudinal hollow (8) opens.

7. A hearing protection earplug according to claim 1, **characterized in that**, laterally next to the outer central longitudinal hollow (8) at least one additional longitudinal hollow (11) is provided that extends parallel to the outer central longitudinal hollow (8) and the central center longitudinal axis (12) into the main body (1).

## Revendications

1. Bouchon d'oreille constitué d'un corps de base (1) à base de mousse, où
a) le corps de base (1) possède un axe médian longitudinal (12) central ainsi qu'une face frontale axiale interne (2) et une face frontale axiale externe (3) en direction de cet axe médian longitudinal (12) central,
b) des évidements (5, 6) intérieurs, augmentant la surface, parmi lesquels l'un s'étend vers l'intérieur en partant de la face frontale interne (2) le long de l'axe médian longitudinal (12) central sous la forme d'un évidement longitudinal central (6) intérieur, sont prévus dans la région de la face interne (2),
c) un évidement longitudinal central externe (8) est prévu, lequel s'étend vers l'intérieur en partant de la face frontale externe (3) le long de l'axe médian longitudinal (12),
d) les évidements longitudinaux centraux interne et externe (6, 8) sont séparés l'un de l'autre par une paroi transversale (7) continue s'étendant perpendiculairement par rapport à l'axe médian longitudinal (12) central, **caractérisé en ce que**
e) les évidements (5, 6) intérieurs augmentant la surface comprennent, au moins partiellement ou par endroits, des évidements d'enveloppe (5) disposés sur une surface d'enveloppe (13) du corps de base (1) et s'étendant dans la direction de l'axe médian longitudinal (12) central.

2. Bouchon d'oreille selon la revendication 1, **caractérisé en ce que** quatre évidements d'enveloppe (5), distribués autour de la surface d'enveloppe (13) de manière régulière, sont prévus.

3. Bouchon d'oreille selon la revendication 1, **caractérisé en ce que** les évidements d'enveloppe (5) sont en forme d'anneaux et s'étendent en partant d'une région latérale de la surface d'enveloppe (13) jusqu'à proximité d'un orifice de l'évidement longitudinal central (6) interne disposé sur la face frontale interne (2).

4. Bouchon d'oreille selon la revendication 1, **caractérisé en ce que** le bouchon d'oreille est conçu d'une seule pièce.

5. Bouchon d'oreille selon la revendication 1, **caractérisé en ce qu'**un élargissement (4) en forme de collerette est prévu dans la région de la face frontale externe (3).

6. Bouchon d'oreille selon la revendication 1, **caractérisé en ce qu'**un évidement de la base (9) est prévu sur la face frontale externe (3), l'évidement longitudinal central (8) externe débouchant dans le fond de base (10) de celui-ci.

7. Bouchon d'oreille selon la revendication 1, **caractérisé en ce que** le au moins un autre évidement longitudinal (11) est prévu latéralement à côté de l'évidement longitudinal central (8) externe, qui s'étend vers l'intérieur du corps de base (1) parallèlement par rapport à l'évidement longitudinal central (8) externe et par rapport à l'axe médian longitudinal (12) central.
